Europäisches Patentamt

European Patent Office

Office européen des brevets

(11) Veröffentlichungsnummer: **0 219 601**
**B1**

# EUROPÄISCHE PATENTSCHRIFT

(45) Veröffentlichungstag der Patentschrift:
03.01.90

(51) Int. Cl.⁴: **C07C 57/03**, C07C 51/08

(21) Anmeldenummer: 86106585.2

(22) Anmeldetag: 15.05.86

(54) Verfahren zur Herstellung von Z-2-Methyl-2-butensäure.

(30) Priorität: 08.10.85 DE 3535889

(43) Veröffentlichungstag der Anmeldung:
29.04.87 Patentblatt 87/18

(45) Bekanntmachung des Hinweises auf die Patenterteilung:
03.01.90 Patentblatt 90/1

(84) Benannte Vertragsstaaten:
CH DE FR GB LI NL

(56) Entgegenhaltungen:
DE-A- 646 929

(73) Patentinhaber: Consortium für elektrochemische
Industrie GmbH, Zielstattstrasse 20,
D-8000 München 70(DE)

(72) Erfinder: Gebauer, Helmut, Dr. Dipl.-Chem.,
Schaffhauser Strasse 18/7, D-8000 München 71(DE)

ACTORUM AG

**Beschreibung**

Die Erfindung betrifft ein neues Verfahren zur Herstellung von Z-2-Methyl-2-butensäure (2-Methylisocrotonsäure, Angelicasäure, 2-Methyl-ciscrotonsäure). Diese Säure kommt in der Natur u.a. als Inhaltsstoff von Angelicawurzelöl vor. Einige ihrer Ester sind als Hauptbestandteile des begehrten Römischen Kamillenöls wichtige Geruchsträger. Die Säure und ihre Ester sind deshalb von Nutzen als Geruchs- und Geschmacksstoffe. Trotz lebhaften Interesses seitens der Parfümeure und Flavoristen ist bisher noch kein ökonomisches Verfahren zur Herstellung der genannten Verbindungen bekannt. Zwar schlägt die EP-A-0112394 eine Synthese von Angelicasäure und deren Estern durch thermische Umlagerung der isomeren Tiglinverbindungen mittels organischer Sulfinsäuren als Katalysator vor, jedoch erfordet dieses Verfahren bereits wertvolle Riechstoffe als Ausgangsmaterialien (Tiglinsäure bzw. Tiglate),darüberhinaus großen apparativen und energetischen Aufwand. Aufgrund der cisoder Z-Konfiguration sind die Z-2-Methyl-2-butensäure und ihre Ester thermisoh instabiler als die entsprechende trans- oder E-Konfiguration (Tiglinsäure, Tiglate): Das Isomerisierungsgleichgewicht liegt daher bei höchstens 10 % Z-Konfiguration im Gemisch. Da die Angelicasäure und ihre Ester einige wenige Grade unterhalb der entsprechenden Tiglinsäure bzw. der Tiglate schmelzen und sieden, können die Isomeren mittels einer aufwendigen Rektifikation getrennt werden. Dieses Verfahren wird dort als "isomerization distillation" bezeichnet.

Es bestand daher die Aufgabe, einen einfacheren und weniger aufwendigen Weg zu finden, aus kostengünstigeren Ausgangssubstanzen in möglichst hohen Ausbeuten die Z-2-Methyl-2-butensäure herzustellen, wobei nur möglichst geringe Gehalte von E-2-Methyl-2-butensäure im Rohprodukt enthalten sein sollen.

Diese Aufgabe konnte nun durch das erfindungsgemäße Verfahren gelöst werden. Gegenstand der Erfindung ist ein Verfahren zur Herstellung von Z-2-Methyl-2-butensäure durch schwefelsaure Hydrolyse von ggf. Verunreinigungen enthaltendem 2-Methyl-2-butennitril bei erhöhter Temperatur und durch anschließende Auskristallisation, das dadurch gekennzeichnet ist, daß man das Nitril bei Temperaturen bis zu 130°C, vorzugsweise bis zu 80° C in 60 - 100, vorzugsweise 75 - 85, insbesondere 75 - 80 gewichts-%ige Schwefelsäure unter Rühren eindosiert, bis ein molares Verhältnis des Nitrils zur Schwefelsäure von 1 : 3 bis 1 : 1 erreicht ist, das Gemisch anschließend unter Rühren auf dieser Temperatur hält, bis sich die Nitrilgruppen vollständig umgesetzt haben, anschließend bis zu einem Schwefelsäuregehalt von 40 - 60 Gewichts-%, vorzugsweise 45 - 55 Gewichts-%, verdünnt, ggf. unter Druck bei Temperaturen von bis zu 130, vorzugsweise bis zu 100° C weiterrührt, bis die Umsetzung zur Säure beendet ist, die organische Phase von der wäßrigen abtrennt, wäscht und anschließend einer Destillation bei Temperaturen bis zu 199° C, vorzugsweise einer Vakuumdestillation bei Temperaturen bis zu 120° C unterwirft, das Destillat abkühlt und die auskristallisierte Z-2-Methyl-2-butensäure abfiltriert.

Das 2-Methyl-2-butennitril, das bei der Herstellung von Adiponitril als großtechnisches Nebenprodukt anfällt und im allgemeinen noch bis zu etwa 40 Gew.-% Verunreinigungen enthalten kann, wird bisher z. B. als Lösungsmittel auf dem Markt angeboten und sogar mangels geeigneter anderer Verwendungsmöglichkeiten verbrannt. Selbst solches technisches Nitril kann er findungsgemäß ein - und umgesetzt werden. Da es hierzu nicht einmal einer vorherigen Reinigung unterworfen werden muß, wird es als Ausgangsmaterial bevorzugt.

Nach dem Datenblatt eines Anbieters besteht es zu 60 - 70 Gew.-% aus 2-Methyl-2-butennitril und enthält im allgemeinen zu 6 - 25 Gew.-% 2-Methyl-3-butennitril, zu 0,5 - 9 Gew.-% Cyclohexan, zu 3 - 7 Gew.-% Vinylcyclohexen, zu 0,1 bis 4 Gew.-% Cyclooctadiene, zu 0 - 2 Gew.-% Butadien, und bis zu 30 ppm Cyanwasserstoff (die Komponenten ergeben insgesamt 100 Gew.-%). Solche verunreinigungshaltige Substanzen werden im allgemeinen als "technisch" bezeichnet. Die bevorzugte Ausgangssubstanz des erfindungsgemäßen Verfahrens wird daher auch in der vorliegenden Anmeldung verallgemeinernd als technisches 2-Methyl-2-butennitril oder technisches Nitril bezeichnet.

Geht man insbesondere von solchem technischen Nitril aus, so ergibt das Verfahren zunächst Gemische, die Z- und E-2-Methyl-2-butensäure enthalten. Bei besonders schonenden Bedingungen, d. h. gemäß der bevorzugten Ausführungsform liegt selbst beim Einsatz von technischem Nitril die Selektivität sehr hoch, und das Isomerenverhältnis liegt dann bei mindestens, vorzugsweise über, insbesondere deutlich über 60 : 40, z. B. über 65 : 35.

Die Lösung der Aufgaben ist insbesondere deshalb, vor allem auch bei Einsatz technischen Nitrils, überraschend, da aus verschiedensten Literaturstellen bekannt ist, daß sich die Z-2-Methyl-2-butensäure und ihre Derivate bereitwillig z.B. im basischen und im sauren pH-Bereich (z. B. in Schwefelsäure) und bei erhöhter Temperatur in die E-Formen umlagern (vgl. z. B. Buckles, Mock und Locatell, Chem. Reviews 55 (1955) S. 659 - 677).

Auf die erfindungsgemäße Weise läßt sich somit ohne großen energetischen und apparativen Aufwand sogar aus technischem Nitril, gemäß der bevorzugten Ausführungsform insbesondere ohne langwierige fraktionierte Destillation Z-2-Methyl-2-butensäure mit einer Reinheit von vorzugsweise über 95 %, insbesondere über 98 %, herstellen. Vor der Fraktionierung durch Kristallisation besteht das in üblicher Weise von Vor- und Nachlauf getrennte Destillat in diesem Fall vorwiegend aus den Z-und E-2-Methyl-2-butensäuren, wobei die E-Säure unvermeidlich aus dem 2-Methyl-3-butennitril entsteht. Vielfach liegt das Verhältnis der Z- zur E-Säure bei 75 : 25 bis 81 : 19.

Stellt man keine so hohen Anforderungen an die Selektivität, so kann die Umsetzung des 2-Methyl-2-butennitrils mit konzentrierterer Schwefelsäure, d. h. solcher mit einer Stärke über 85 %, durchgeführt werden. Dabei tritt aber bereits teilweise Umlagerung der Z-2-Methyl-2-butensäure zur E-Säure ein. Gleiches gilt, wenn die Temperaturen oberhalb des bevorzugten Bereiches gehalten werden. Führt man andererseits die Umsetzung des Nitrils bei Temperaturen unter z. B. 70° C, beispielsweise bei 20 - 70° C, oder mit stark verdünnter Säure durch, so verlängern sich die Reaktionszeiten zum Teil stark. Das Verfahren ist dann nicht mehr so wirtschaftlich, wird schließlich sogar unwirtschaftlich. Vorzugsweise werden daher die Temperaturbereiche von 70 - 80° C und 70 - 100°C in den beiden Reaktionsstufen eingehalten. Während der Eindosierung des Nitrils in die Schwefelsäure kann die Temperatur vorzugsweise auch niedriger gehalten werden, z. B. unter 60° C, insbesondere z. B. bei 30 - 55° C.

Vorzugsweise werden dem Reaktionsgemisch Polymerisationsinhibitoren zugesetzt, z. B. ein Gemisch aus Hydrochinon und Zinksulfat. Als besonders vorteilhaft hat sich der Zusatz von 0, 1 bis 1 Mol% solcher Inhibitoren, bezogen auf eingesetztes Nitrils erwiesen. Entsprechend ihrer Fähigkeit, Polymerisationsreaktionen einzugehen, kann die Z-2-Methyl-2-butensäure auch als (Co-)-Monomer bei radikalischen Polymerisationen eingesetzt werden.

Das folgende Beispiel dient der weiteren Erläuterung der Erfindung (die Prozentangaben beziehen sich auf das Gewicht). Es wurde ein Roh-Nitril nachstehender Zusammensetzung (durch Gaschromatographie bestimmt) eingesetzt:

| Bestandteil | % |
|---|---|
| 2-Methyl-2-butennitril | 63 |
| 2-Methyl-3-butennitril | 20 |
| Cyclohexan | 5,5 |
| Vinylcyclohexen | 6,6 |
| Cyclooctadien | 1,9 |
| Butadien | 1,1 |
| nicht definiert | 1,9 |

<u>Beispiel</u>

In einer emaillierten 360 l-Technikumsapparatur mit Kühl- und Rühreinrichtung wurden 120 kg 80%ige Schwefelsäure zusammen mit jeweils 100 g Hydrochinon und Zinksulfat vorgelegt. Unter Kühlen und Rühren ließ man innerhalb zwei Stunden 81 kg (99 l) Rohnitril zulaufen, wobei die Temperatur auf 50 - 55° C gehalten wurde.
Nach 24 Stunden Reaktionszeit bei 80° C wurden 80 l Wasser zugegeben und weitere 32 Stunden nunmehr bei 100° C gerührt. Nach Zugabe von 60 l Toluol werden die Phasen getrennt und die organische Phase der Destillation zugeführt.
Nach Entfernen der Niedrigsieder gingen im Siedebereich von Kp$_{12}$ 83 - 99° C 63,5 kg Produktgemisch, bestehend aus 80,5 Angelica- und 19,5 % Tiglinsäure über, aus dem durch Kristallisation reine Angelicasäure abgetrennt werden konnte.
Rückstand der Destillation: 18 kg.

**Patentansprüche**

1. Verfahren zur Herstellung von Z-2-Methyl-2-butensäure durch schwefelsaure Hydrolyse von 2-Methyl-2-butennitril bei erhöhter Temperatur und durch anschließende Auskristallisation, **dadurch gekennzeichnet,** daß man das Nitril bei Temperaturen bis zu 130° C in 60 - 100 gewichts-%ige Schwefelsäure unter Rühren eindosiert, bis ein molares Verhältnis des Nitrils zur Schwefelsäure von 1 : 3 bis 1 : 1 erreicht ist, das Gemisch anschließend unter Rühren auf diesen Temperaturen hält, bis sich die Nitrilgruppen vollständig umgesetzt haben, anschließend bis zu einem Schwefelsäuregehalt von 40 - 60 Gew.-% verdünnt, bei Temperaturen von bis zu 130° C weiterrührt, bis die Umsetzung zur Säure beendet ist, die organische Phase von der wäßrigen abtrennt, wäscht und anschließend einer Destillation bei Temperaturen bis zu 199° C unterwirft, das Destillat abkühlt und die auskristallisierte Z-2-Methyl-2-butensäure abfiltriert.

2. Verfahren nach Anspruch 1, **dadurch gekennzeichnet,** daß die Temperatur beim Eindosieren des Nitrils und Umsetzen der Nitrilgruppen höchstens bei 80° C, in der Folge bei bis zu 100° C und während der Destillation unter Vakuum bei höchstens 120° C gehalten wird.

3. Verfahren nach Anspruch 1 oder 2, **dadurch gekennzeichnet,** daß dem Reaktionsgemisch Polymerisationsinhibitoren in einer Konzentration von 0,1 bis 1 Mol%, bezogen auf eingesetztes Nitril, zugesetzt werden.

**Claims**

1. Process for the preparation of Z-2-methyl-2-butenoic acid by sulphuric acid hydrolysis of 2-methyl-2-butene-nitrile at elevated temperature and by subsequent crystallization, characterized in that the nitrile is metered into 60–100% strength by weight sulphuric acid at temperatures up to 130°C, while stirring, until a molar ratio of the nitrile to the sulphuric acid of 1:3 to 1:1 is reached, the mixture is subsequently kept at this temperature, while stirring, until the nitrile groups have reacted completely, and then the mixture is diluted to a sulphuric acid content of 40–60% by weight, continuing to stir at temperatures of up to 130° until the conversion into the acid has ended, the organic phase is separated off from the aqueous phase, washed and then subjected to distillation at temperatures up to 199°C, the distillate is cooled and the Z-2-methyl-2-butenoic acid which has crystallized out is filtered off.

2. Process according to Claim 1, characterized in that the temperature is kept at not more than 80°C during metering in of the nitrile and reaction of the nitrile groups, at up to 100°C during the subsequent period and at not more than 120°C during the distillation in vacuo.

3. Process according to either of Claims 1 and 2, characterized in that polymerization inhibitors are added to the reaction mixture in a concentration of 0.1 to 1 mol %, based on the nitrile employed.

## Revendications

1. Procédé de fabrication du Z-2-méthyl-2-buténoïque par hydrolyse à chaud en milieu d'acide sulfurique du 2-méthyl-2-buténonitrile puis séparation par cristallisation, procédé caractérisé en ce que l'on introduit progressivement le nitrile sous agitation, à des températures pouvant s'élever jusqu'à 130°C, dans de l'acide sulfurique à 60–100% en poids, jusqu'à un rapport molaire du nitrile à l'acide sulfurique compris entre 1:3 et 1:1, puis on maintient le mélange sous agitation à ces températures jusqu'à ce que les groupes nitrile soient totalement transformés, on le dilue ensuite à une teneur en acide sulfurique de 40 à 60% en poids, on poursuit l'agitation à des températures pouvant s'élever jusqu'à 130°C jusqu'à ce que la transformation en acide soit terminée, on sépare la phase organique de la phase aqueuse, on la lave puis on la soumet à une distillation jusqu'à 199°C, on refroidit le distillat et on en sépare par filtration le Z-2-méthyl-2-buténoïque qui a cristallisé.

2. Procédé selon la revendication 1, caractérisé en ce que l'on maintient la température au maximum à 80°C au cours de l'addition du nitrile et de la transformation des groupes nitrile, on la maintient ensuite jusqu'à 100°C et pendant la distillation sous vide au maximum à 120°.

3. Procédé selon la revendication 1 ou 2, caractérisé en ce que l'on ajoute au mélange réactionnel des inhibiteurs de polymérisation à une concentration de 0,1 à 1 mol % par rapport au nitrile employé.